Europäisches Patentamt

European Patent Office

Office européen des brevets

(19)

(11) Publication number: **0 400 847**
**A2**

(12) **EUROPEAN PATENT APPLICATION**

(21) Application number: **90305388.2**

(22) Date of filing: **18.05.90**

(51) Int. Cl.⁵: **G01N 33/49, //G01N11/12**

(30) Priority: **27.05.89 GB 8912277**

(43) Date of publication of application:
**05.12.90 Bulletin 90/49**

(84) Designated Contracting States:
**AT BE CH DE DK ES FR GB GR IT LI LU NL SE**

(71) Applicant: **FISONS plc**
**Fison House Princes Street**
**Ipswich Suffolk IP1 1QH(GB)**

(72) Inventor: **Goddard, Nicholas John**
**143 Greenfields**
**Earith, Cambridgeshire PE17 3QZ(GB)**
Inventor: **Lowe, Christopher Robin**
**The Limes, Hempstead**
**Saffron Walden, Essex CB10 2PW(GB)**

(74) Representative: **Wright, Robert Gordon McRae**
**FISONS plc 12 Derby Road**
**Loughborough Leicestershire LE11 0BB(GB)**

(54) **Apparatus for detecting change in fluid state of a liquid.**

(57) An apparatus for detecting a change in the fluid state of a liquid comprises a sample chamber (23) containing an agitator (29), means for displacing the agitator (29) from its resting position and then releasing it such that it can return to its resting position under the influence of gravity, and sensor means (31) for detecting the return of the agitator (29) to its resting position.

The apparatus is particularly useful in the measurement of blood clotting times, and is especially advantageous in that it allows accurate measurements to be made across a wide range of clotting times. In this case the walls of the chamber are conveniently coated with a measured quantity of thromboplastin.

Fig.7.

This invention relates to an apparatus and method for detecting a change in the fluid state of a liquid sample, and more particularly for determining the time of coagulation of samples of blood or blood plasma.

A number of devices and methods for the determination of blood clotting time have been described previously.

In one such device (British Patent No 1235589), the blood under test is held in a test-tube which contains a steel ball. Magnets on either side of the tube hold the ball static at a fixed position while the tube is subjected to reciprocating motion along its long axis. When a clot forms the ball is displaced, this displacement being detected by an optical sensor. Other devices utilising analogous principles of operation are described, for example, in British Patent No 1508946 and US Patent No 3520659.

Prior art devices of the kind described above suffer from the disadvantage that the driving force exerted on the ball relative to the test-tube renders the device rather insensitive to slow changes in viscosity as occur in samples with long clotting times. In addition, the devices are of relatively complex construction and involve large numbers of moving parts. Also, the sample volumes required are relatively large. Furthermore, measured volumes of sample must be transferred to the sample jar or tube, eg by pipetting. Thus, the devices require a considerable degree of expertise on the part of their users.

Developments in the diagnostic field are increasingly directed towards devices intended for the carrying out of clinical tests (such as measurements of blood clotting time, eg the prothrombin time) not in the laboratory, but in the doctor's surgery or even in the patient's own home. Such devices must be reliable yet of simple construction; must be inexpensive to manufacture and, above all, must be easy to operate. The disadvantages of the prior art devices referred to above therefore render them particularly unsuitable for routine use in such environments.

We have now devised a method and device for detecting changes in the fluid state of a liquid sample, eg the coagulation of a blood sample, which overcomes or substantially mitigates these disadvantages.

Thus, according to the invention there is provided an apparatus for detecting a change in the fluid state of a liquid, which comprises a sample chamber containing an agitator, means for displacing the agitator from its resting position and then releasing it such that it can return to its resting position under the influence of gravity, and sensor means for detecting the return of the agitator to its resting position.

The apparatus of the present invention is advantageous in that is of relatively simple construction, involves few moving parts, is inexpensive to manufacture and is easy to operate. As such, the apparatus is suitable for the carrying out of clinical tests (such as measurements of blood clotting time, eg the prothrombin time) not in the laboratory, but in the doctor's surgery or even in the patient's own home. Furthermore, the large degree of automation which is possible results in improved accuracy and reproducibility of results. Also, the sample volumes required are relatively small. The apparatus is particularly useful in the measurement of blood clotting times, and is especially advantageous in that it allows accurate measurements to be made across a wide range of clotting times.

The chamber may have any convenient shape, but is preferably arranged, in use, to be substantially vertical.

The optimum volume of the chamber will depend principally on the quantity of material available for analysis. For the determination of blood clotting time, for instance, where it is desired to perform analyses on single drops of blood the chamber volume is preferably rather low, typically $20\mu l$ or less.

The cross-sectional profile of the chamber is preferably such as to facilitate egress of air as the liquid enters the chamber and to permit the flow of liquid past the agitator as the agitator moves within the chamber. Particularly when the volume of the chamber is low, the internal surface of the chamber is also preferably wettable to facilitate flow of the liquid into the chamber.

The agitator may have any convenient form but is preferably a sphere.

The dimensions of the agitator will naturally depend on the size of the chamber in which it is located. However, for the determination of blood coagulation times in a chamber of volume less than $20\mu l$, it is preferred to use a spherical agitator of diameter approximately 2.5mm. The chamber is then preferably less than 5mm, typically approximately 3mm in length so that the distance travelled by the agitator is only about 0.5mm.

Preferably, the agitator is ferromagnetic and the means for displacing the agitator from its resting position comprises a magnet. For example, the means for displacing the agitator may comprise an electromagnet located above the chamber and means of alternately energising and deenergising the magnet so as to draw the agitator from the bottom of the chamber to the top and then release it. The optimum frequency with which the magnet is energised will vary with the nature of the liquid under investigation, the volume of the chamber etc. However, in the determination of blood coagulation times with a chamber and agitator of the form and dimensions described above, a frequency of about 10Hz is found to be appropriate.

The sensor means may have various forms. It may, for example, be a magnetic sensor capable of detecting changes in magnetic field, eg a coil or a Hall effect transducer. Alternatively, the sensor means may be optical, eg a light source and photodetector (eg a light emitting diode and a photodiode) arranged on opposite sides of the chamber. We prefer, however, to employ a sensor capable of detecting the impact of the agitator on the base of the chamber. Examples of such sensors include acoustic sensors, eg one or more microphones, and piezoelectric sensors arranged at the bottom of the chamber such that impact of the agitator on the sensor generates a voltage. Such sensors are advantageous when compared with, for example, optical sensors in that they may be located further from the base of the chamber.

Since it is generally the time taken for the fluid state of the sample to change that is the parameter of interest, the apparatus according to the invention is preferably operably linked to some suitable timer. The timer is preferably triggered automatically at the appropriate starting point. Often it is the entry of the liquid into the chamber which is the appropriate starting point. There may therefore be provided a sensor capable of detecting the presence of test liquid in the chamber and triggering the timer. The liquid presence sensor may have various forms, eg it may be an optical sensor or a conductimetric sensor. The timer may also be switched off automatically when a change in the motion of the agitator is detected.

Where the sensor means used to monitor the motion of the agitator and to detect a change in that motion operates by detecting pulses, eg an interruption in an optical pathway caused by the agitator passing through an optical sensor, and those pulses are separated by known time intervals, then the timer may comprise a counter capable of accumulating the number of such pulses occurring before the change in fluid state occurs and converting that number to a time. The interval between pulses, or the interval between the release of the agitator and the next following pulse, may also correlate with the viscosity of the liquid under test.

For many analyses it is necessary to mix the sample under test with a reagent. For instance, in the measurement of prothrombin time a blood sample is mixed with thromboplastin. The reagent is conveniently coated, eg by freeze-drying, onto the internal walls of the chamber or onto the surface of the agitator. Alternatively, the reagent may be coated on the walls of an inlet leading into the sample chamber.

The measurement may be carried out at particular pre-determined temperature, for example blood clotting time measurements will generally be carried out at normal body temperature, ie 37°C. The chamber may therefore be thermostatted at 37°C and blood at that temperature may be introduced directly from the patient. Alternatively, the apparatus may include means of thermostatting the sample at the desired temperature prior to mixing with any necessary reagents and the beginning of the measurement. There may also be provided automatic mixing means adapted to mix the sample with the reagent(s) once the desired temperature has been reached. For example, there may be provided an intermediate sample reservoir separated from the chamber by a barrier, eg a membrane, which is removed, eg the membrane may be punctured, once the contents of the reservoir reach the desired temperature.

The apparatus is preferably provided with output means to indicate the result of the test, eg an alphanumeric display and/or a printer.

In a preferred embodiment of the invention, particularly useful for the determination of blood coagulation time, the chamber and agitator form part of a disposable sample cell, eg of plastics material, which has formed within it an inlet for the liquid to be tested as well as the chamber containing the agitator and reagent with which the liquid is to be mixed. The reagent may, for example, be freeze-dried on to the walls of the chamber. Where optical sensor means are employed to detect the return of the agitator to its resting position or to detect the presence of liquid in the chamber, the block is preferably transparent. The sample cell is used in association with an apparatus containing magnetic means capable of displacing the agitator from its resting position and then releasing it, and sensor means capable of detecting the return of the agitator to its resting position.

In such a sample cell the surface of the the chamber is preferably coated with a wetting agent so as to facilitate flow of liquid into the chamber. The chamber also performs a metering function, the quantity of reagent contained within it being appropriate to the volume of liquid which flows into the chamber and the particular test to be performed.

A particularly preferred form of such disposable sample cell comprises a generally flat unit provided with a sample well containing a measured quantity of reagent and a ferromagnetic agitator, the agitator being retained in the chamber by a ferromagnetic member which, in use, serves as a polepiece for an electromagnet.

In many cases it may be necessary to adjust the result of the measurement by means of a correction factor to take account of variations, eg between batches of reagent. The apparatus may therefore be provided with means of entering correction factors or other calibration data. This may conveniently be done by means of a bar code reader.

According to another aspect of the invention, there is provided a method of detecting a change in the fluid state of a liquid, which comprises

a) introducing a sample of the liquid into a metering chamber containing an agitator and a quantity of reagent for the liquid appropriate to the volume of the chamber,

b) displacing the agitator from its resting position, and

c) allowing the agitator to return to its resting position under the influence of gravity.

A preferred embodiment of the invention will now be described by way of illustration only with reference to the accompanying drawings in which

Figure 1 is a perspective view of a device for measuring prothrombin time,

Figure 2 is a perspective view of a disposable sample cell for use in association with the device of Figure 1,

Figure 3 is a side view of the disposable sample cell of Figure 2,

Figure 4 is a plan view of the disposable sample cell of Figure 2,

Figure 5 is a sectional view along the line V-V in Figure 4,

Figure 6 is a bottom view of the disposable sample cell of Figure 2, and

Figure 7 is a schematic view of certain components of the device of Figure 1 with the disposable sample cell of Figure 2 in place.

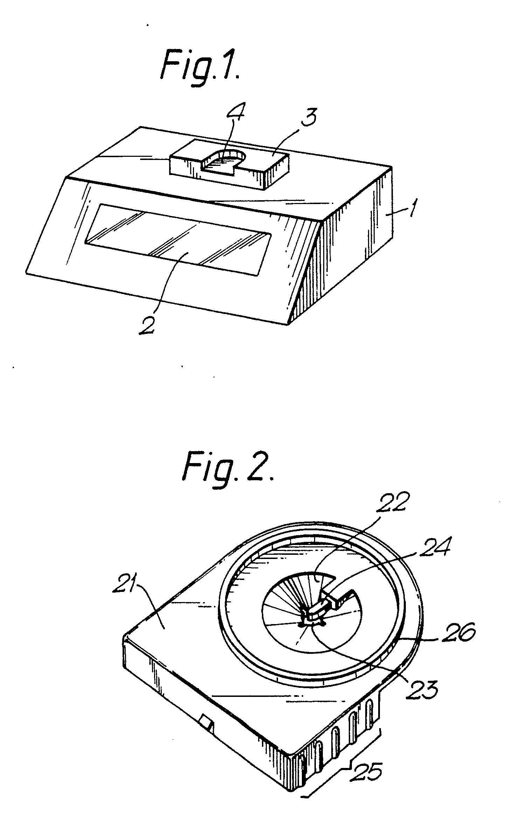

Referring first to Figure 1, a device for measuring prothrombin time comprises a housing (1) provided with an alphanumeric display (2) and a thermostatted block (3) which is maintained at a temperature of 37°C. The block (3) has a slot (4) to receive the disposable sample cell depicted in Figures 2 to 6.

The disposable sample cell (21), as shown in Figure 2, is of generally flat configuration and is provided, near one end, with a shallow funnel (22) leading into a generally cylindrical chamber (23). The upper, open end of the chamber (23) is partially covered by an iron polepiece (24) which extends through the side of the funnel (22) to the underside of the cell (21). The cell (21) is provided with finger grips (25) for ease of handling and a circular rim (26) to reduce the risk of spillage of blood introduced into the funnel (22).

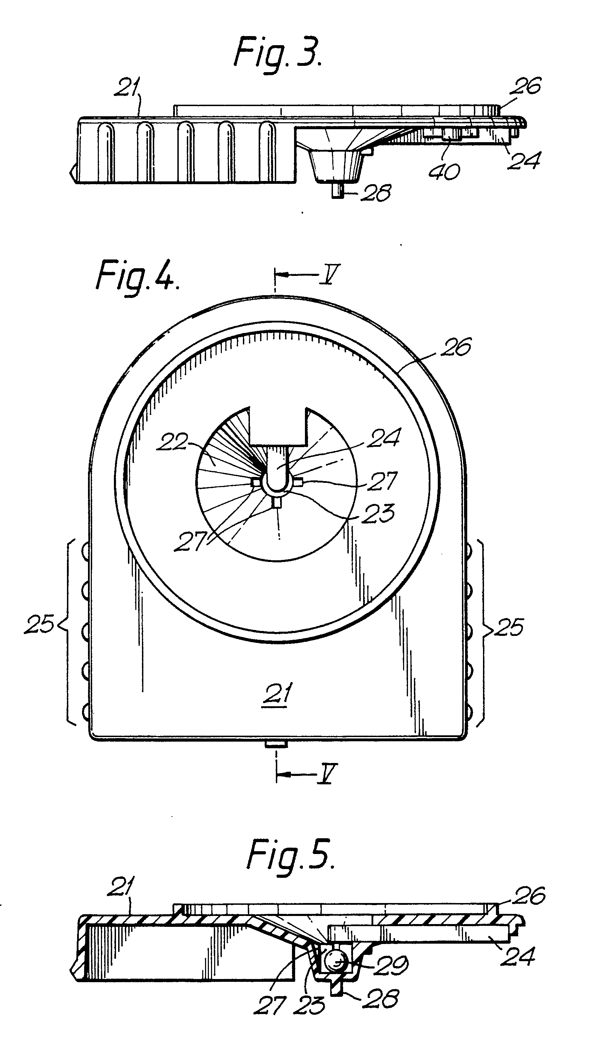

As can be seen from Figures 3 and 5, the underside of the base of the chamber (23) is provided with a downwardly extending solid pin (28). The chamber (23) includes a steel ball (29) of diameter 2.4mm which is retained within the chamber (23) by the polepiece (24) such that the vertical travel of the ball (29) is limited to about 0.6mm. Also provided on the underside of the cell (21) are two downwardly depending cylindrical lugs (40,41) to facilitate correct location of the cell (21) within the slot (4).

The internal surface of the chamber (23) is coated with dried thromboplastin reagent and, as is most clearly seen from Figure 4, the chamber (23) is provided with three radially extending channels (27) to facilitate egress of air when the chamber (23) fills with blood.

Embedded in the sides of the chamber (23) are a pair of diametrically opposed conductimetric electrodes (51,52) which are used to detect the presence of blood in the chamber (23).

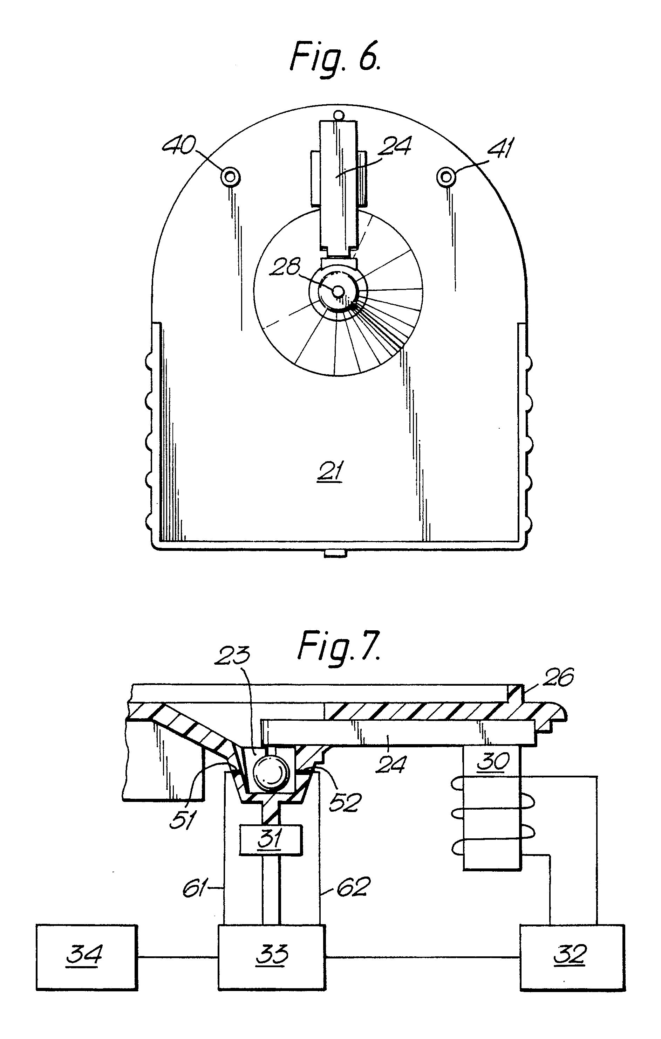

The sample cell (21) is customarily supplied in a sealed, sterile sachet which is removed immediately before the cell (21) is to be used. The disposable sample cell (21) is then located in the slot (4) on the thermostatted block (3). When the cell (21) is correctly located on the block (3) the lugs (40,41) actuate a switch (not shown) to indicate the presence of the cell (21) in the device. As can be seen from Figure 7, when the cell (21) is in position, the polepiece (24) contacts an electromagnet (30) which is connected to a switchable power supply unit (32) which is in turn connected to, and controlled by, a microprocessor (33). The electrodes (51,52) are connected to the microprocessor (33) by connectors (61,62) and the pin (28) bears against a piezoelectric transducer (31) connected to the microprocessor (33). The microprocessor (33) is also connected to the alphanumeric display (2).

After sufficient time for the temperature of the chamber (23) to become equilibrated at 37°C, the patient's fingertip is punctured and a drop of blood expressed. The fingertip is then inserted into the funnel (22) thereby transferring the drop of blood into the funnel (22) and the chamber (23). The presence of blood in the chamber (23) is detected by the conductimetric electrodes (51,52) and initiates the measuring sequence. The electromagnet (30) is energised at a frequency of 10Hz by the power supply (32). Each time the electromagnet (30) is energised, the ball (29) is drawn to the top of the chamber (23); when the electromagnet (30) is switched off, the ball (29) returns to the bottom of the chamber (23).

The impact of the ball (29) on the base of the chamber (23) is detected by the piezoelectric transducer (31), the impact being transmitted to the transducer (31) by the pin (28).

This sequence continues until the ball (29) fails to return to its resting position due to clot formation within the chamber (23).

The time between initiation of the reaction and clot formation is stored, any necessary correction or calibration factors applied and the result (the prothrombin time) displayed on the alphanumeric display (2). The sample cell (21) may then be removed from the slot (4) in the thermostatted block (3) and discarded.

**Claims**

1. An apparatus for detecting a change in the fluid state of a liquid, which comprises a sample chamber containing an agitator, means for displacing the agitator from its resting position and then releasing it such that it can return to its resting position under the influence of gravity, and sensor means for detecting the return of the agitator to its resting position.

2. An apparatus according to Claim 1, wherein the volume of the chamber is 20μl or less.

3. An apparatus according to Claim 1 or Claim 2, wherein the agitator is ferromagnetic and the means for displacing the agitator from its resting position comprises a magnet.

4. An apparatus according to any one of the preceding claims, wherein the sensor means is capable of detecting the impact of the agitator on the base of the chamber.

5. An apparatus according to Claim 4, wherein the sensor means comprises a piezoelectric transducer.

6. An apparatus according to any one of the preceding claims, wherein the chamber contains a measured quantity of a reagent.

7. An apparatus according to Claim 6, wherein the walls of the chamber are coated with a measured quantity of thromboplastin.

8. An apparatus according to any one of the preceding claims, which comprises

   a) an apparatus containing magnetic means capable of displacing the agitator from its resting position and then releasing it, and sensor means capable of detecting the return of the agitator to its resting position, and

   b) a disposable sample cell which has formed within it an inlet for the liquid to be tested as well as the chamber containing the agitator and reagent with which the liquid is to be mixed.

9. A disposable sample cell comprising a generally flat unit provided with a sample well containing a measured quantity of reagent and a ferromagnetic agitator, the agitator being retained in the chamber by a ferromagnetic member which, in use, serves as a polepiece for an electromagnet.

10. A method of detecting a change in the fluid state of a liquid, which comprises

   a) introducing a sample of the liquid into a metering chamber containing an agitator and a quantity of reagent for the liquid appropriate to the volume of the chamber,

   b) displacing the agitator from its resting position, and

   c) allowing the agitator to return to its resting position under the influence of gravity.

Fig.1.

Fig. 2.

# Fig. 3.

# Fig.4.

# Fig.5.

# Fig. 6.

# Fig. 7.